(19) Europäisches Patentamt
European Patent Office
Office européen des brevets

(11) Publication number: 0 361 239 A2

(12) # EUROPEAN PATENT APPLICATION

(21) Application number: 89117129.0

(51) Int. Cl.5: A61K 31/565

(22) Date of filing: 15.09.89

The title of the invention has been amended (Guidelines for Examination in the EPO, A-III, 7.3).

(30) Priority: 26.09.88 US 248851

(43) Date of publication of application: 04.04.90 Bulletin 90/14

(84) Designated Contracting States: AT BE CH DE ES FR GB GR IT LI NL SE

(71) Applicant: STERLING DRUG INC.
90 Park Avenue
New York New York(US)

(72) Inventor: Keister, Don Mark
R.D. 1 Box 426
West Sand Lake New York 12196(US)

(74) Representative: Baillie, Iain Cameron et al
c/o Ladas & Parry Isartorplatz 5
D-8000 München 2(DE)

(54) **Use of epostane for increasing ovulation rate in swine.**

(57) The method of increasing ovulation rate and litter size in swine which comprises administering to a gilt or sow during the middle to late part of the estrus cycle an amount of epostane sufficient to increase follicular recruitment and thereby increase the number of ova released by the ovaries and, after insemination, to increase the number of viable fertilized ova implanted in the uterus and, after gestation, to increase the number of live piglets delivered is described.

EP 0 361 239 A2

The invention relates to a method of increasing fecundity in swine using epostane.

Epostane is the United States Adopted Name (1986 USAN and the USP dictionary of drug names, 1961-1985 cumulative list) for (4α,5α,17β)-4,5-epoxy-3,17-dihydroxy-4,17-dimethylandrost-2-ene-2-carbonitrile having the structural formula

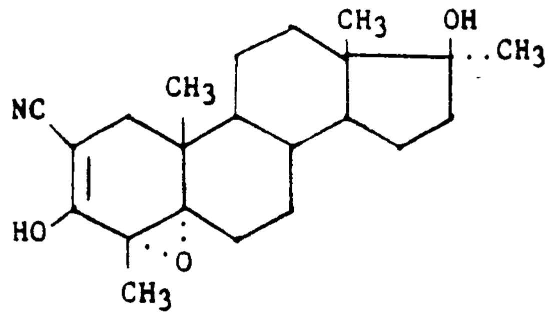

Formula I

and having utility as an interceptive (pregnancy disrupting) agent.

Christiansen U.S. Pat. 4,160,017 issued July 3, 1979 describes epostane as the product of part (f) of EXAMPLE 1, that is, 4α,5α-epoxy-17β-hydroxy-4,17-dimethyl-3-oxoandrostane-2α-carbonitrile having the structural formula

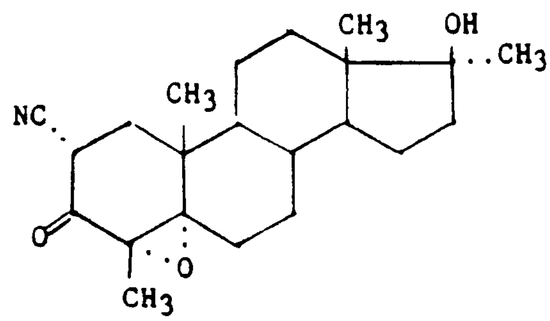

Formula II,

which represents the keto form of epostane. Formula I represents the enol form. The patent shows the interceptive utility of epostane in the rat and the monkey.

A Ledger et al. (Journal of Steroid Biochemistry, vol. 17, no. 3, p. xci, abst. 271, 1982) paper entitled THE SUCCESS OF LABOUR INDUCED BY PROGESTERONE WITHDRAWAL IN PREGNANT SHEEP describes the use of epostane "to induce labour in sheep during late pregnancy".

The master of science in animal science degree thesis of Jeffrey A. Blackwell (New Mexico State University, Las Cruces, New Mexico, 1984) entitled REPRODUCTIVE PERFORMANCE OF EWES TREATED WITH AN INHIBITOR OF PROGESTERONE SYNTHESIS describes the effects of epostane medication at day 10 of the estrus cycle in cycling ewes. At a dose of 50 mg. serum progesterone levels and recycling time increased, conception rate was not adversely affected, and the number of lambs produced increased.

A second Ledger et al. paper (Journal of Endocrinology, vol. 105, pp. 227-233, 1985) describes the "effects of an inhibitor of 3β-hydroxysteroid dehydrogenase (epostane) on uterine activity and cervical softening...in eight sheep during late pregnancy". Uterine activity was observed to be "similar to that seen in the final stages of labor" and the cervix was observed "to have softened considerably".

The Arthur Walpole Memorial Lecture for the Society for the Study of Fertility by Robert Webb (University of Aberdeen, 1985) describes increasing ovulation rate and production of lambs in ewes with epostane.

Fowden et al. (J. Reprod. Fert., Suppl., vol. 35, pp. 539-545, 1987) shows the failure of intravenous epostane (up to 900 mg. per animal) to induce parturition in the pregnant mare near term.

Miner European Patent Application 256,224 published February 24, 1988 describes the method of inducing estrus in the early luteal phase of the normally cycling cow using epostane.

Embryo transfer is a useful technique in domestic animals including swine for improving the breed, especially to prevent and/or control disease, whereby embryos are collected from an oviduct of an inseminated female and transferred to the uterus of a surrogate female. Heretofore only pregnant mare serum gonadotropin has been used to increase ovulation rate for this purpose in swine.

Reproductive efficiency in swine is governed by the number of farrowings in a given period of time and by litter size. The former can be maximized by good herd management, but aside from maintaining a healthy herd the latter has been heretofore subject to natural control. An exception is the use of antibodies to androstenedione (FECUNDIN, Glaxo) to increase litter size.

The presently described and claimed invention provides a method of increasing ovulation rate and litter size pharmacologically instead of immunologically.

## SUMMARY OF THE INVENTION

In a first aspect the invention is the method of increasing ovulation rate in swine which comprises administering to a gilt or sow during the middle to late part of the estrus cycle an amount of epostane sufficient to increase follicular recruitment and thereby increase the number of ova released by the ovaries.

In a second aspect the invention is the method of increasing litter size in swine which comprises administering to a gilt or sow during the middle to late part of the estrus cycle an amount of epostane sufficient, after insemination, to increase the number of viable fertilized ova implanted in the uterus and, after gestation, to increase the number of live piglets delivered.

## DETAILED DESCRIPTION OF THE INVENTION INCLUSIVE OF THE PREFERRED EMBODIMENTS

The normal estrus cycle in the pig is 21 days in length. "[M]iddle to late part of the estrus cycle" means from approximately day 10 to approximately day 21. "[F]ollicular recruitment" refers to the number of ovarian follicles developed on the ovary in preparation for ovulation. "[O]vulation rate" is the number of ova released by the ovaries per gilt or sow per cycle. In carrying out a clinical test of the invention ovulation rate is determined by counting the number of corpora lutea on the ovaries at necropsy following sacrifice at 30 days after the first insemination.

On the several factors which affect litter size including ovulation rate, fertilization rate and embryo survival rate, ovulation rate is considered most important. Unlike the cow or the ewe or the mare, which usually ovulates a single ovum, the gilt or sow ovulates multiple ova at estrus and has a normal ovulation rate of about 15. The maximum possible ovulation rate is known to be about 18 in the gilt and about 23 in the primiparous sow. There thus appears to be a significant potential for increasing ovulation rate from the normal by pharmacological intervention. Increasing fertilization rate or embryo survival rate by pharmacological intervention does not appear possible. The goal of increasing litter size therefore appears achievable if the chosen pharmacological intervention increases ovulation rate without decreasing fertilization rate or embryo survival rate. It is known that although uterine crowding may contribute to fetal loss during the latter part of gestation, it is not a major factor limiting litter size during the first 25 days thereof. Accordingly, in carrying out a clinical test of the invention, by determining the pregnancy rate, that is, by counting the number of fertilized ova implanted in the uterus per gilt or sow (viable implants) at necropsy following sacrifice at 30 days after the first insemination, a measure of litter size is determined wherein the factor of fetal loss due to uterine crowding is eliminated. Two further advantages are gained by carrying out the clinical test in this manner. Possible fetal loss due to adverse effect of the drug can be assessed so that dosing can be adjusted accordingly, and the clinical test can be conducted in less time and more economically.

Any amount of epostane sufficient to effect an increase in ovulation rate and litter size can be used. The preferred dose is in the range of 0.1-10 mg./kg. per day. The epostane can be prepared for administration in any pharmaceutically acceptable oral, vaginal, rectal or parenteral dosage form. The oral dosage form can be solid or liquid and can be granules, capsule, tablet, solution, suspension or emulsion. The vaginal or rectal dosage form is preferably the suppository. The parental dosage form can be solution, suspension or emulsion. An oral dosage form is preferred. The preferred oral dosage form is granules in the feed or capsule.

A clinical test of the invention was carried out using prepubertal Yorkshire gilts, each weighing about 245 lb. (111 kg.). Estrus cycling was stimulated by exposure to adult boars without allowing mounting. Day 1 of the estrus cycle was taken as the day on which a gilt would first stand to be mounted. Nine groups of twelve gilts each were used. One group received sham medication and served as controls. Four groups were medicated with epostane on days 10-21 of the cycle and four groups were medicated with epostane

on days 15-21 of the cycle. Four dose levels of epostane were used, each for each of the two dosing schedules: 0.625 mg./kg., 1.25 mg./kg., 2.5 mg./kg. and 5.0 mg./kg. per day. The epostane was administered orally as granules in a small amount of feed provided that any epostane-containing feed not accepted by the gilt be replaced by epostane capsule(s) administered orally. Estrus behavior with adult boars was checked twice (at about 0800 and 1800 hours) daily. Each gilt was artificially inseminated at 24 hours and again at 36 hours after onset of the second estrus and sacrificed 30 days after insemination. Ovulation rate and pregnancy rate were determined by examination of the ovaries and uterus at necropsy. Means and standard deviations were calculated for each dose group. These results and the range of ovulation rate or pregnancy rate values and number of gilts (N) of each dose group are set forth in the following two tablets. In both tables N is less than 12 in some instances because not all gilts completed the test.

Table I

| Ovulation Rate | | |
|---|---|---|
| Epostane Dose Mg./Kg. per Day | Mean Number of Corpora Lutea ± Standard Deviation (Range) (N) | |
| | Days 10-21 Treatment | Days 15-21 Treatment |
| 0 (Controls) | 15.4 ± 2.3 (14-22)(12) | — |
| 0.625 | 19.4 ± 3.4 (14-24)(12) | 17.7 ± 2.8 (14-22)(12) |
| 1.25 | 21.8 ± 3.9 (17-30)(11) | 19.5 ± 3.1 (16-26)(10) |
| 2.5 | 17.9 ± 2.6 (15-21)(9) | 21.5 ± 2.7 (18-26)(12) |
| 5.0 | 21.4 ± 5.5 (12-28)(10) | 23.3 ± 4.7 (17-32)(12) |

Table II

| Pregnancy Rate | | |
|---|---|---|
| Epostane Dose Mg./Kg. per Day | Mean Number of Viable Implants ± Standard Deviation (Range) (N) | |
| | Days 10-21 Treatment | Days 15-21 Treatment |
| 0 (Controls) | 12.1 ± 2.5 (9-15)(8) | — |
| 0.625 | 14.0 ± 3.9 (9-19)(9) | 11.0 ± 4.0 (4-17)(10) |
| 1.25 | 10.4 ± 3.2 (5-16)(8) | 11.9 ± 3.4 (8-17)(8) |
| 2.5 | 14.3 ± 3.5 (11-18)(3) | 13.6 ± 4.5 (9-21)(8) |
| 5.0 | 9.8 ± 3.1 (7-14)(6) | 12.6 ± 3.9 (5-16)(9) |

The data were subjected to analysis of variance for overall comparison of dose groups followed by linear regression analysis to test for dose response.

With regard to the data of Table I the analysis of variance showed a significant difference in mean ovulation rate among dose groups ($p = 0.0001$). The linear regression analysis showed a significant dose response within the 15-21 day dose groups ($p = 0.0001$) but not within the 10-21 day dose groups. Based on the estimated regression lines the mean ovulation rates of all but the dose group given 0.625 mg./kg. of epostane per day on days 15-21 of the cycle are significantly greater than that of the control group ($p < 0.002$).

With regard to the data of Table II the analysis of variance did not show a significant difference in pregnancy rate among dose groups (p = 0.28) and the linear regression analysis did not show a significant dose response within either the 10-21 day dose groups (p = 0.10) or the 15-21 day dose groups (p = 0.24)

Although the above-described clinical test did not show a statistically significant increase in pregnancy rate, since it did show a statistically significant increase in ovulation rate, I believe that a statistically significant increase in pregnancy rate can be achieved by reducing the number of treatment days from twelve or even seven, shifting the dosing schedule away from the end of the cycle, and choosing one of the lower epostane doses, for example, by administering 1.25 mg./kg. of epostane per day on days 13-18 of the cycle.

## Claims

1. A method of increasing the ovulation rate in swine which comprises administering to a gilt or sow during the middle to late part of the estrus cycle an amount of epostane sufficient to increase follicular recruitment and thereby increase the number of ova released by the ovaries.

2. A method according to claim 1, wherein the amount of epostane is in the range of 0.1-10 mg./kg. per day.

3. A method according to claim 2, wherein the amount of epostane is from about 0.625 mg./kg. per day to about 5.0 mg./kg. per day.

4. A method according to any one of the preceding claims, wherein the epostane is administered orally.

5. A method according to any one of the preceding claims, wherein the epostane is administered on some or all of days 10-21 of the estrus cycle.

6. A method according to claim 5, wherein the epostane is administered on each of days 15-21 of the estrus cycle.

7. The use of epostane for preparing a product useful for administering to a gilt or sow during the middle to late part of the estrus cycle to increase follicular recruitment and thereby increase the number of ova released by the ovaries.